# EUROPEAN PATENT SPECIFICATION

(11) **EP 4 233 853 B1**
(45) Date of publication and mention of the grant of the patent: **19.08.2026**
(21) Application number: 23164354.5
(22) Date of filing: 30.09.2016
(51) Int. Cl.: A61K 31/164, A61K 31/728, A61P 19/02, A61P 19/04, A61K 31/16

(54) **ADELMIDROL FOR USE IN THE TREATMENT OF ARTICULAR CHONDROPATHIES**
ADELMIDROL ZUR VERWENDUNG IN DER BEHANDLUNG ARTIKULÄRER CHONDROPATHIEN
ADELMIDROL POUR L'UTILISATION DANS LE TRAITEMENT DE CHONDROPATHIES ARTICULAIRES

(30) Priority: 30.10.2015 IT UB20154849
(43) Date of publication of application: 30.08.2023
(62) Document number(s) of the earlier application(s) in accordance with Art. 76 EPC: 21153063.9 / 3 827 819; 16191868.5 / 3 162 365
(73) Proprietor: EPITECH GROUP S.p.A., 20144 Milano (MI) (IT)
(72) Inventor: DELLA VALLE, Francesco, deceased (IT); DELLA VALLE, Maria Federica, I-20144 Milan (IT); CUZZOCREA, Salvatore, I-20144 Milan (IT)
(74) Representative: Long, Giorgio

(56) References cited:
- EP-A1- 2 965 765
- DI PAOLA ROSANNA ET AL: "Adelmidrol, in combination with hyaluronic acid, displays increased anti-inflammatory and analgesic effects against monosodium iodoacetate-induced osteoarthritis in rats", ARTHRITIS RESEARCH & THERAPY, vol. 18, no. 1, 1 December 2016 (2016-12-01), XP093052494, DOI: 10.1186/s13075-016-1189-5
- BOILEAU CHRISTELLE ET AL: "The Peroxisome Proliferator-Activated Receptor ? Agonist Pioglitazone Reduces the Development of Cartilage Lesions in an Experimental Dog Model of Osteoarthritis In Vivo Protective Effects Mediated Through the Inhibition of Key Signaling and Catabolic Pathways", ARTHRITIS & RHEUMATISM, vol. 56, no. 7, 7 July 2007 (2007-07-07), pages 2288 - 2298, XP093197060, DOI: 10.1002/art.22726
- DANIELE DE FILIPPIS ET AL: "Adelmidrol, a palmitoylethanolamide analogue, reduces chronic inflammation in a carrageenin-granuloma model in rats", JOURNAL OF CELLULAR AND MOLECULAR MEDICINE, vol. 13, no. 6, 1 June 2009 (2009-06-01), pages 1086 - 1095, XP055159615, ISSN: 1582-1838, DOI: 10.1111/j.1582-4934.2008.00353.x

## Description

### Technical field of the invention

The present invention relates to a pharmaceutical composition containing Adelmidrol.

### Background art

The musculoskeletal system is a preferential target for inflammatory processes even arisen in other tissues than in said system (such as articular inflammatory processes related to chronic Inflammatory Bowel Diseases (IBDs). At the articular level, the inflammatory process gradually becomes chronic and non-resolving. In association with the non-resolving intra-articular inflammation, some alterations occur in the delicate homeostasis of the cartilage tissue mainly consisting of chondrocytes and fibroblasts, resulting in the gradual local destruction of articular cartilages, and there are alterations of the delicate interactions between the articular cartilage and the metabolism of the subchondral bone, up to erosion processes of the subchondral bone. Such disease processes, which are highly disabling for those whom they affect, are referred to as articular chondropathies or joint chondropathies.

Articular chondropathies are progressive degenerative diseases of the articular cartilages generally, although not exclusively, related to individuals' aging, and even when the pathogenesis is of different origin (traumatic, iatrogenic, dismetabolic origin, etc.) or associated with an inflammatory process mainly related to a different organ not belonging to the osteoarticular system (as in the case of inflammatory bowel diseases), aging strongly contributes to the progression of the disease.

Over the past decade, the international research reserved utmost importance to the molecular mechanisms which control the homeostatic balance of the articular cartilage tissue; the results in terms of protection of cartilage tissues have been very poor so far, if not actually absent.

Increasing attention has recently been paid to the expression and function of a nuclear receptor of great importance in regulating the process of re-synthesis and degradation of cartilages; in particular, the functional significance of PPAR-gamma receptor (Peroxisome Proliferator-Activated Receptor gamma) in ensuring the homeostatic balance of the articular compartment has been explored in detail. It has been particularly shown how experimental animals genetically lacking PPAR-gamma spontaneously develop an intense process of degradation of articular cartilages caused by hypo-cellularity of chondrocytes, fibrogenesis of cartilages, and subchondral bone remodeling; in essence, it is now confirmed that PPAR-gamma is a critical regulator of the homeostasis of cartilage tissues and its decreased activity in the joints significantly contributes to accelerate the development of homeostatic alterations of the articular cartilage tissue and therefore the degradation thereof which is not sufficiently compensated by re-synthesis processes.

The expression of PPAR-gamma is low in the articular cartilages of human subjects suffering from joint inflammations accompanied by degradation of the articular cartilages, compared to healthy subjects, and in dogs, synthetic agonists of PPAR-gamma significantly reduce the expression of inflammatory/catabolic mediators and protect the articular cartilages against degradation. For this reason, we tried to test clinically synthetic agonists of PPAR-gamma in subjects with underway cartilage degradation; so far, such molecules, in addition to not negligible side effects, have shown non-specific effects for this receptor.

The role of PPAR-gamma receptor in tissue protection of the colonic mucosa was also recently highlighted by the technical literature, and the therapeutic potentials of agonists of the same receptor during chronic inflammatory bowel diseases (IBDs) (Crohn's disease, ulcerative colitis) and irritable bowel syndrome (IBS) were evaluated.

In addition, it has been shown how the PPAR-gamma receptor is strongly involved in systemic diseases characterized by pathological fibrotic processes of the connective tissue, and in particular in Systemic Sclerosis, especially of the skin and lung.

Finally, it has been shown that the PPAR-gamma receptor plays an exceptionally important role in several diseases related to the eye, of both the ocular surfaces and the retina.

Adelmidrol is a derivative of azelaic acid (azelaic acid diethanolamide) which belongs, as regards the structure and the mechanism of action, to the family of Aliamides: molecules, with the progenitor Palmitoylethanolamide (PEA), the properties of which, as known for some time, mainly pass through the sub-modulation of the mast cells residing in the various tissue districts (ALIA, Autacoid Local Injury Antagonism mechanism).

The publication Boileau Christelle ET AL: Arthritis & Rheumatism, vol. 56, no. 7, 7 July 2007 (2007-07-07), pages 2288-2298, discloses the effect of a PPAR gamma agonist, pioglitazone, in the treatment of osteoarthritis in dogs.

The publication Daniele De Filippis ET AL: Journal of Cellular and Molecular Medicine, vol. 13, no. 6, 1 June 2009 (2009-06-01), pages 1086-1095 discloses the effect of Adelmidrol in the reduction of chronic inflammation in a carrageenin-granuloma model in rats.

### Summary of the invention

Therefore, there is a need to have a highly safe, specific agonist of the PPAR gamma receptor for the treatment of diseases mediated by such a mechanism of action.

Therefore, it is an object of the present invention to provide Adelmidrol for use in the treatment of diseases characterized by articular cartilage degeneration caused by insufficient agonism of the PPAR-gamma receptor, as set out in the appended claims.

Further features and advantages of the invention will become apparent from the following description of preferred embodiments, given by way of non-limiting example.

### Brief description of the drawings

**Figure 1****. (A)** Images obtained by a stereomicroscope on the state of erosion of the interposed cartilage in the right knee of each experimental group: control, MIA, Adelmidrol 150µg/25µL i.a.. The analysis was performed 21 days after the induction of the disease. **(B)** Effect of the treatment with Adelmidrol 150µg/25µL i.a. on the erosive lesion of the interposed cartilage upon injection of MIA, compared to MIA and control groups treated with the solvent;
**Figure 2** **(for information only).** Microscope image showing the tissue damage in the bowel caused by the administration of DNBS and the protection given by the administration of Adelmidrol;
**Figure 3** **(for information only).** A chart showing the results of a visual assessment of the tissue damage in the bowel caused by the administration of DNBS and the protection given by the administration of Adelmidrol;
**Figure 4****.** A chart showing the results on MPO activity upon treatment with DNBS or DNBS + Adelmidrol;
**Figure 5** **(for information only).** A chart showing the weight variation of animals treated with bleomycin and Adelmidrol;
**Figure 6** **(for information only).** A chart showing the difference between the wet lung weight and the lung weight following water removal in animals treated with bleomycin and Adelmidrol and not treated animals;
**Figures 7** **and** **8** **(for information only).** Charts showing the distribution of inflammatory cells in BAL in animals treated with bleomycin and Adelmidrol and not treated animals;
**Figure 9** **(for information only).** A chart showing the dosage of TNF-α and IL1β in animal samples treated with bleomycin and Adelmidrol and not treated animal samples.

### Detailed description of the invention

The present invention relates to Adelmidrol for use in the treatment of diseases sensitive to the specific agonism of the PPAR-gamma receptor, wherein said diseases are articular chondropathies.

The invention stems from the surprising discovery that Adelmidrol is able to act effectively as a specific agonist of the PPAR-gamma receptor. From such a finding it is apparent that Adelmidrol is capable of determining a pharmacological action in the cartilage degradation processes in validated animal models of inflammation.

It has been seen that, if Adelmidrol is infiltrated into the joint or administered systemically, it shows therapeutic activity by controlling the pathological phenomena associated with intra-articular inflammatory process (chondroprotection, antifibrogenic effect of cartilage and sub-chondral bone).

Finally, it has been seen that Adelmidrol acts on the PPAR-gamma receptor in a specific manner, having confirmed that, even in the joint, Adelmidrol is not able to act as an agonist of PPAR-alpha.

Adelmidrol is the symmetric diethanolamide of azelaic acid of formula:

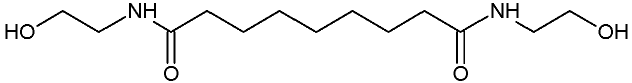

Adelmidrol is a product which can be easily obtained by condensation of two moles of ethanolamine with one mole of azelaic acid under conventional conditions for amide bond formation.

### EXPERIMENTAL SECTION

### a) Effect of systemically administered Adelmidrol on inflammation

The experimental model of carrageenan-induced rat paw edema was used.

The experimental model of carrageenan-induced paw edema was induced by sub-plantarily injecting a solution of carrageenan (containing 50 µl of sterile saline containing 1% carrageenan) in the animal's right paw (rat) .

The foot volume was measured at specific time intervals by means of a plethysmometer (Ugo Basile, Milan, Italy). The increase in plantar volume was assessed as the difference between the value obtained at the specific time intervals and the volume at baseline (time 0) measured immediately before the administration of carrageenan.

The rats (10 animals per group) were divided into the following experimental groups:
- CAR + physiological solution;
- CAR + Adelmidrol (3 mg/kg); Adelmidrol was administered i.p. when injecting CAR;
- CAR + Adelmidrol (10 mg/kg); Adelmidrol was administered i.p. when injecting CAR.

The results obtained, expressed as a percentage with respect to the edema produced by carrageenan alone, are shown in Table 1:

| **TABLE 1** | **0 min.** | **30 min** | **1 hr** | **2 hr** | **3 hr** | **4 hr** | **5 hr** |
|---|---|---|---|---|---|---|---|
| Carrageenan (CAR) | 100.0 | 100.0 | 100.0 | 100.0 | 100.0 | 100.0 | 100.0 |
| CAR+ Adelmidrol (3 mg/kg) | 101.2 | 86.7 | 71.9 | 72.3 | 69.8 | 67.2 | 67.4 |
| CAR+ Adelmidrol (10 mg/kg) | 102.6 | 81.6 | 68.7 | 63.8 | 53.6 | 49.5 | 49.5 |

The treatment with Adelmidrol resulted in a reduction of the carrageenan-induced edema with a dose-dependent action.

### b) Effect of intra-articularly administered Adelmidrol on inflammation, pain and erosion of articular cartilages in an animal model of monosodium iodoacetate (MIA)-induced articular lesion

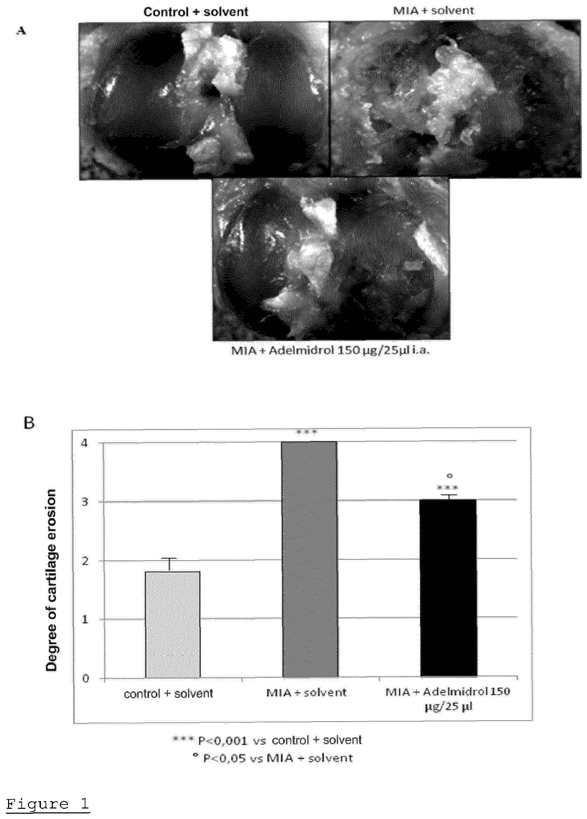

A microscopic analysis of the lesion related to the articular cartilage was performed by comparing the erosive lesion of the articular cartilage between the following groups: animals not treated with MIA, animals treated with MIA + solvent, and animals treated with MIA + solvent added with Adelmidrol. **Figure 1A** shows the most representative images of each group, obtained by means of a stereomicroscope. In the image corresponding to the animal damaged through MIA, an extensive cartilage erosion is apparent, which also allows to distinguish the subchondral bone, unlike the animal control whose joint is in excellent condition. The tibial plateau of an animal treated with Adelmidrol 150µg/25µL i.a. shows a clear protection against cartilage erosion. Each image was given a score from 0 to 4 (0 = normal; 4 = maximum degree of injury severity) expressing the articular cartilage lesion in terms of increasing severity *(*Janusz MJ et al. Ostearthritis and Cartilage 2001;9:751-760*),* as described by Guingamp et al. (Guingamp C. et al. Arthritis & Rheumatism 1997; Vol 40 No 9:1670-1679).

The quantitative analysis (**Figure 1B**) shows that, as known from the literature (*Guingamp C et al. 1997* and *Janusz MJ et al. 2001, above),* the animals damaged through MIA show a high degree of cartilage erosion, compared to control animals. The analysis shows that Adelmidrol 150µg/25µL i.a. is able to noticeably slow down the cartilage erosion in a statistically significant manner.

This experiment proves that Adelmidrol is able to noticeably slow down the cartilage erosion while preserving the morphological and functional integrity of the joint.

### c) Evidence that the effect of Adelmidrol is caused by agonism on the PPAR-gamma receptor

In order to show that the protective action of Adelmidrol on pain is related to the agonism on the peroxisome proliferator-activated receptor gamma (PPAR-gamma), the effect of the PPAR-gamma receptor antagonist, GW9662, was studied in the same experimental model of carrageenan (CAR)-induced rat paw edema. The experimental model of carrageenan-induced rat paw edema is as shown above. In this experiment, the effect was assessed by measuring the residence time of the animal's paw on the hot plate, thus obtaining percentage data with respect to the control treated with carrageenan alone.

The rats (10 animals per group) were divided into the following experimental groups:
- CAR + physiological solution;
- CAR + GW9662 (1 mg/kg) + Adelmidrol (10 mg/kg); GW9662 was administered i.p. 30 min before injecting CAR and Adelmidrol (i.p.) when injecting CAR;
- CAR + Adelmidrol (10 mg/kg); Adelmidrol was administered i.p. when injecting CAR.

The results obtained, expressed as a percentage with respect to the edema produced by carrageenan alone, are shown in Table 2:

| **TABLE 2** | **0 min.** | **30 min** | **1 hr** | **2 hr** | **3 hr** | **4 hr** | **5 hr** |
|---|---|---|---|---|---|---|---|
| Carrageenan (CAR) | 100.0 | 100.0 | 100.0 | 100.0 | 100.0 | 100.0 | 100.0 |
| CAR+GW9662 (1 mg/kg) +Adelmidrol (10 mg/kg) | 105.2 | 96.2 | 102.4 | 104.1 | 111.3 | 114.1 | 112.5 |
| CAR+Adelmidrol (10 mg/kg ip) | 107.4 | 98.5 | 123.2 | 134.2 | 146.8 | 197.7 | 175.0 |

Pretreatment with GW9662 antagonized the effect of Adelmidrol thus substantially abolishing the protective action thereof.

### d) Evidence that the effect of Adelmidrol is not caused by possible agonism on the PPAR-alpha receptor

In order to verify a possible involvement of PPAR-alpha receptor, the edema induced by carrageenan in the manners described above was carried out on PPAR-alpha knock-out mice (PPAR-α-WT and KO).

PPAR-α-WT and KO mice (10 animals per group) were divided into the following experimental groups:
- CAR + physiological solution;
- CAR + Adelmidrol (10 mg/kg); Adelmidrol was administered i.p. when injecting CAR.

The absence of the PPAR-α receptor did not alter the protective action of Adelmidrol.

The results obtained, expressed as a percentage with respect to the edema produced by carrageenan alone, are shown in Table 3:

| **TABLE 3** | **0 min.** | **30 min** | **1 hr** | **2 hr** | **3 hr** | **4 hr** | **5 hr** |
|---|---|---|---|---|---|---|---|
| Carrageenan (CAR) in PPAR-α-WT and KO mice | 100.0 | 100.0 | 100.0 | 100.0 | 100.0 | 100.0 | 100.0 |
| CAR+Adelmidrol (10 mg/kg) in PPAR-α-WT and KO mice | 102.4 | 81.9 | 69.2 | 63.9 | 55.6 | 51.4 | 50.3 |

Adelmidrol also shows activity on the carrageenan-induced edema in mice genetically lacking the PPAR-alpha receptor; therefore, the effect of Adelmidrol is not dependent on the PPAR-alpha receptor.

### e) Effect of orally administered Adelmidrol on the bowel tissue lesion induced by locally administering DNBS (dinitrobenzene sulfonic acid) (for information only)

Rats were anesthetized with ethyl ether in order to ensure the animals' waking up after 4-5 minutes. 60 mg/mL DNBS was dissolved in 50% ethanol in water. An amount of 0.25 mL of alcoholic solution (containing 15 mg of DNBS) was instilled to each animal, using an 8 cm long intrarectal catheter made of silicone so as to reach the spleen.

During the experiment, the possible ejection of the solution from the rectum when the animal awoke was carefully monitored, taking it into account in the final assessment. The assessment of the bowel lesion was performed by taking, upon sacrifice of the animal, the entire bowel which was adequately washed with physiological solution and immediately examined under a microscope.

Adelmidrol was administered per os through a gastric cannula in an amount of 10 mg/kg dissolved in aqueous solution containing 2% CMC (carboxymethylcellulose).

The bowel tissue lesion was quantized through microscope inspection by two different observers. The lesion quantization was carried out by assigning a numeral score from 0 to 8 according to the following scheme:
0 = no lesion
1 = localized hyperemia without ulcers
2 = linear ulcers without evident inflammation
3 = linear ulcers with inflammation on one side only
4 = two or more major sites of inflammation and ulceration with more than 1 cm extension
from 5 to 8 = 1 point was added for each centimeter of ulceration in addition the initial 2 cm.

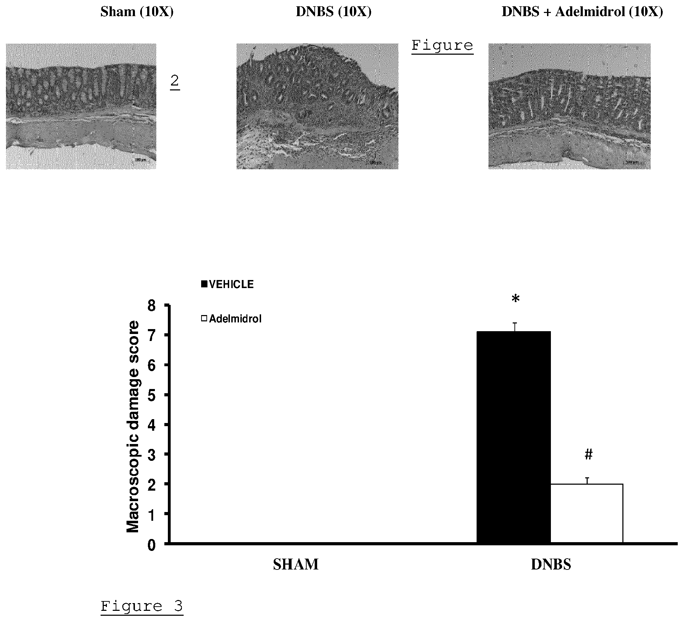

The results obtained are documented by the microscopic images in Figure 2 and given in the chart in Figure 3.

Adelmidrol determines an important, significant protection of the colonic mucosa due to the mechanism of action (PPAR-gamma) found in accordance with the literature.

The determination of the activity of the enzyme myeloperoxidase (MPO), which is considered an important marker of neutrophil activity in the bowel, fully confirms the effect of Adelmidrol on the colonic mucosa. The test was carried out by the method described in the literature (Morampudi V. et al. Journal of Visualized Experiments- February 2014;84:1-8) on DBNS-treated animals, not treated animals, or animals orally treated with Adelmidrol (10 mg/kg).

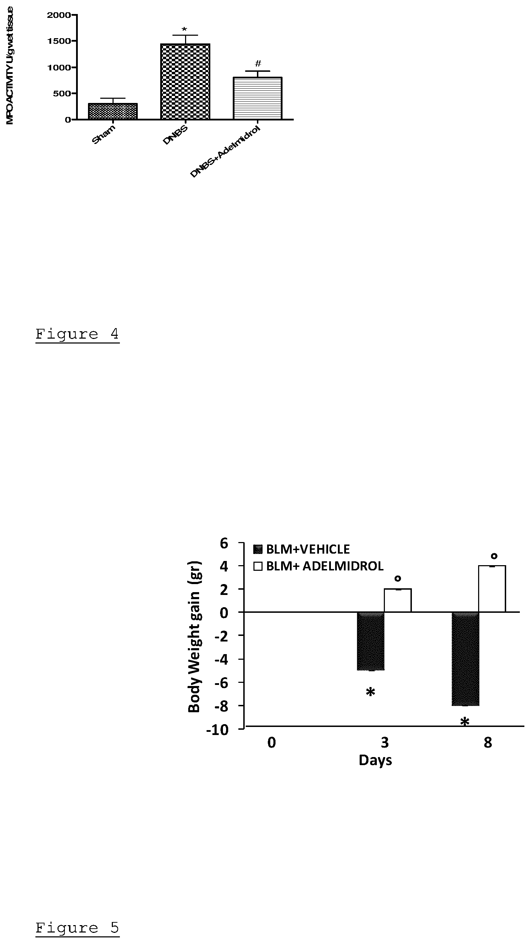

The results are shown in the chart in Figure 4. The results clearly show the activation of neutrophils following the administration of DNBS and the antiinflammatory effect on the bowel tissue after the administration of Adelmidrol.

### f) Effect of Adelmidrol on lung tissue lesion induced by administering bleomycin sulfate (for information only)

### Animals used

CD1 male mice ((CD1(ICR) mice (25-35 g; Harlan Nossan, Italy) were accommodated in a controlled environment and fed with standard food and water.

### Lung lesion induction

Using a catheter (tracheotomy tube), the mice received a single intratracheal instillation of physiological solution (0,9%) or saline containing bleomycin sulfate (0.1 U/mouse) in a final volume of 100 µL, and the liquid was immediately followed by 300 µL of air in order to also ensure the distribution thereof in the distal airways.

### Bronchoalveolar lavage (BAL)

Seven days after the induction of fibrosis, the mice were sacrificed and their trachea was immediately cannulated with an intravenous catheter made of polyethylene (Neo Delta Ven. 2, Delta Med, Viadana, Italy) equipped with a 1 mL 24-gauge syringe. The lungs were washed once with 0.5 mL of D-PBS (GIBCO, UK). In 95% of mice, the recovery volume exceeds 0.4 mL. The BAL fluid was centrifuged at 800 rpm, the surfactant was removed and the cell pellet was resuspended in PBS. The number of cells in the BAL fluid was obtained by counting on a hemocytometer in the presence of Trypan Blue. Cytospins were prepared from the cells resuspended in PBS. The slides were dried in dry air and then colored with Diff-Quick Set Stain (Diff-Quick, Baxter Scientific, Miami, FL). Using the optical microscopy, a total of 400 cells was examined from each sample in randomly chosen fields.

### Measurement of lung edema

The weight of the wet lungs was calculated after 28 days upon injecting bleomycin by means of careful excision of the lung from the adjacent tissues. The lungs were exposed for 48 hours at 180 °C and the dry weight was then assessed. The lung water content was calculated as wet weight-to-dry weight ratio.

### Myeloperoxidase activity assay

The activity of myeloperoxidase (MPO) - an indicator of polymorphonuclear leukocytes (PMNs) accumulation - was determined as previously described in lung homogenates (Mullane KM et al. J of Pharmacological Methods 1985; 14:157-167). The rate of change in absorbance was measured spectrophotometrically at 650 nm. The MPO activity was defined as the amount of enzyme which degrades 1 µmole of peroxide/min at 37 °C and was expressed in milliunits per gram of wet tissue.

### Remarks

All procedures related to the treatment of animals were performed in accordance with the guidelines approved by the Institutional Committee (IACUC) following the guidelines of the Association for Assessment and Accreditation of Laboratory Animal Care (AAALAC). While being monitored, the animals were inspected for any adverse effects produced by the lung lesion. An assessment of the water and food consumption and consequently of the body weight (analyzed daily by weighing the animals) was carried out (*Mullane KM et al, 1985, supra*).

### Results:

### a) Weight change in the animals (for information only)

The animals belonging to the group treated with bleomycin(BLM)+ inert vehicle and those belonging to that treated with BLM and Adelmidrol in an inert vehicle were weighed, and the weight change was calculated on the 3^{rd} and 8^{th} day after the start of the treatment with BLM. The results are shown in the chart in Figure 5.

The animals treated with BLM and Adelmidrol show a significant body weight gain, while those treated with BLM and inert vehicle alone show remarkable weight losses.

### b) Wet weight-to-dry weight ratio of the lung (for information only)

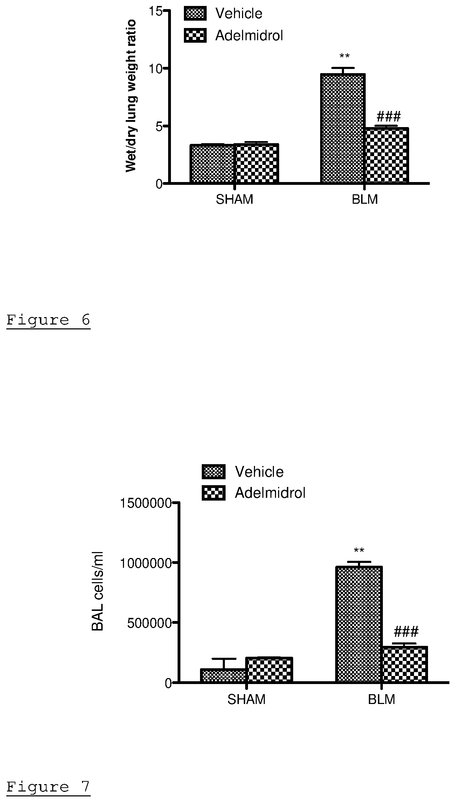

In order to assess the degree of the existing lung edema, at the end of the experiment, even in untreated animals, the comparison of the wet lung weight to the lung weight after water removal was carried out. The results are shown in the chart in Figure 6.

The difference in lung weight between fresh organ and dried organ in the animals treated with BLM and Adelmidrol is considerably lower than that of the animals treated with BLM and inert vehicle alone; Adelmidrol does not change the difference in lung weight between fresh organ and dried organ in animals not treated with BLM.

### c) Change of inflammatory cells in the bronchoalveolar lavage (BAL) fluid (for information only)

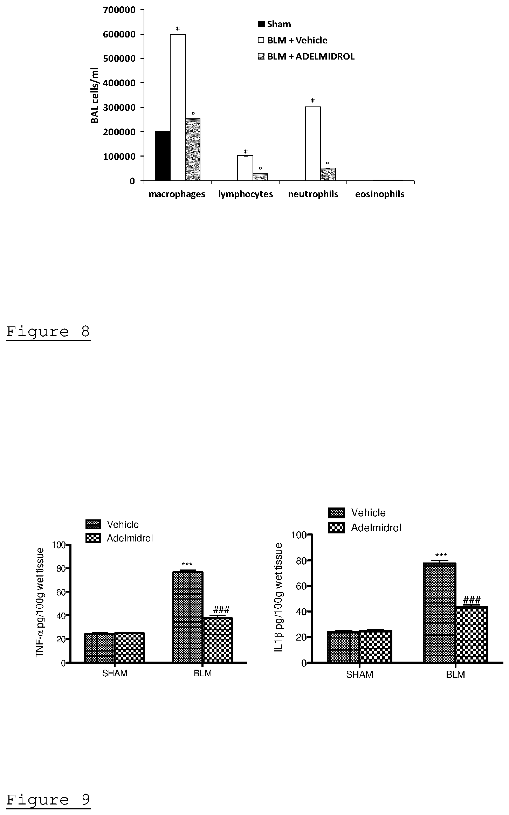

The bronchoalveolar lavage (BAL) is carried out to assess the amount and origin of the inflammatory cells recruited in the lung following the treatment with BLM. The results obtained are shown in the charts in Figures 7 and 8.

The administration of BLM determines a very important increase in inflammatory cells in the lung and in particular in macrophages, neutrophils, and to a lesser extent in lymphocytes, while the eosinophil count remains unchanged. The treatment with Adelmidrol reduces the number of macrophages, neutrophils and lymphocytes in a very significant manner.

### d) Dosage of inflammatory cytokines TNF-α and IL1β (for information only)

TNF-α and IL1β are also regarded as the biochemical markers of inflammation and fibrosis of the lung. The results obtained are shown in the chart in Figure 9.

Significant increases in both TNF-α and IL1β are found in the lung of BLM-treated animals as compared to non BLM-treated animals. Adelmidrol considerably reduces the levels of both inflammatory cytokines.

It is thus apparent that Adelmidrol can be used in the treatment of diseases sensitive to the agonism of the PPAR-gamma receptor in humans or animals, in particular of the following diseases:
- articular chondropathies of mechanical, toxic, iatrogenic, degenerative origin or associated with inflammatory phenomena mainly related to organs and tissues not belonging to the osteoarticular system.

Adelmidrol is formulated for parenteral administration by injection for infiltration into a joint.

The formulations for injection may be in the form of a single dose, e.g. in ampoules, with an added preservative. The compositions may be in such a form as suspensions, solutions or emulsions in oily or aqueous vehicles, and may contain formulary agents such as suspending agents, stabilizers and/or dispersants. Alternatively, the active ingredient may be in powder form to be reconstituted before use with a suitable vehicle, e.g. sterile water.

According to the present invention, the Adelmidrol dose suggested for the administration to a human with a body weight of about 70 kg is from 1 mg to 7 g or from 10 mg to 700 mg of the active ingredient per unit dose. The unit dose may be administered 1 to 4 times per day, for example. It is worth noting that continuous dosage variations could be required depending on the age and weight of the patient and even on the severity of the clinical condition to be treated. The precise dose and route of administration, within the scope of the present invention as defined by the claims, will ultimately be at the discretion of the attending physician or veterinarian.

The formulations according to the invention may be prepared according to conventional methods, such as those described in Remington's Pharmaceutical Sciences Handbook, Mack Pub. Co., N.Y., USA, 17th edition, 1985.

The invention will now be further described by means of the following examples of formulations.

### Examples of formulations:

### Example 1 - Preloaded syringes with sterile solution for intra-articular infiltration

A 2 mL preloaded syringe contains:
- Adelmidrol 50 mg
- Isosmotic solvent buffered to pH 7.0 q.s. to 2 mL.

### Example 2 - Sterile ampoule for infiltration in small movable joints

A 1 mL vial contains:
- Adelmidrol 20 mg
- Isosmotic solvent buffered to pH 7.0 q.s. to 1 mL.

### Example 3 - Sterile vial for intra-articular infiltration

A 5 mL vial contains:
- Adelmidrol 40 mg
- Hyaluronic acid sodium salt 20 mg
- Isosmotic solvent buffered to pH 7.0 q.s. to 5 mL.

### Example 4 - Syrup for oral use (for information only)

A 100 mL bottle contains:
- Adelmidrol 4000 mg
- Carboxymethylcellulose 3000 mg
- Noveon AA1 150 mg
- Sweetener 10 mg
- Distilled water q.s. to 100 mL.

### Example 5 - Granules for veterinary use (for information only)

100 g of granules contain:
- Adelmidrol 6000 mg
- Sorbitol powder 400 mg
- Sucrose palmitate 20 mg
- Appetite stimulant for animals mg 30.

### Example 6 - Tablets for oral use for human or veterinary use (for information only)

One tablet contains:
- Adelmidrol 300 mg
- Microcrystalline cellulose 100 mg
- Croscarmellose sodium 70 mg
- Polyvinylpyrrolidone 10 mg
- Magnesium stearate 4 mg.

### Example 7 - Hard gelatin capsules for oral use for human or veterinary use (for information only)

One capsule contains:
- Adelmidrol 400 mg.

### Example 8 - Soft gelatin capsules (for information only)

One capsule contains:
- Adelmidrol 300 mg
- Vegetable oil 200 mg
- Soy lecithin 50 mg.

### Example 9 - Thick solution for rectal use (for information only)

A 10 mL micro-enema contains:
- Adelmidrol 300 mg
- Resveratrol 100 mg
- Transcutol q.s. to 10 mL.

### Example 10 - Sterile, apyrogenic injectable solution

A 2 mL ampoule contains:
- Adelmidrol 200 mg
- Phosphate buffer pH 7.0 q.s. to 2 mL.

### Example 11 - Cream for external local application on joints (for information only)

100 g of cream contain:
- Adelmidrol 2000 mg
- PEG5 plant sterols 4.5 g
- Stearic acid 3.0 g
- Cetostearyl alcohol 3.0 g
- Glyceryl monostearate 1.5 g
- Carbopol 940 0.40 g
- 2,4-Dichlorobenzyl alcohol 0.15 g
- Bronopol 0.05 g
- Water q.s to 100.00 g.

### Example 12 - Powder for inhalation (for information only)

1.0 g of powder for inhalation contains:

| | |
|---|---|
| - Micronized Adelmidrol (particle size 2÷8 microns) | 0.60 g |
| - Ultrafine lactose | 0.40 g. |

### Example 13 - Aerosol solution (for information only)

1.0 mL of aerosol solution contains:

| | |
|---|---|
| - Adelmidrol | 0.40 g |
| - Polysorbate 80 | 0.10 g |
| - Distilled water q.s. to | 1.00 mL. |

### Example 14 - Solution for eye drops (for information only)

10 mL of solution for eye drops contain:

| | |
|---|---|
| - Adelmidrol | 0.20 g |
| - Hyaluronic acid sodium salt | 0.010 g |
| - Na2HPO4 | 0.0096 g |
| - NaH2PO4 | 0.00284 g |
| - Sodium Chloride | 0.070 g |
| - Distilled water q.s. to | 10.00 mL. |

### Example 15 - Solution for eye drops (for information only)

10 mL of solution for eye drops contain:

| | |
|---|---|
| - Adelmidrol | 0.15 g |
| - Ultra-micronized PEA | 0.0030 g |
| - Hyaluronic acid sodium salt | 0.010 g |
| - Na2HPO4 | 0.0096 g |
| - NaH2PO4 | 0.00284 g |
| - Sodium Chloride | 0.070 g |
| -Distilled water q.s. to | 10.00 mL. |

### Example 16 - Ophthalmic ointment (for information only)

10 g of ophthalmic ointment contain:

| | |
|---|---|
| - Adelmidrol | 0.10 g |
| - Liquid paraffin | 1.00 g |
| - White petrolatum | 8.90 g. |

## Claims

1. Adelmidrol for use in the treatment of diseases sensitive to the specific agonism of the PPAR-gamma receptor in humans or animals wherein said diseases are articular chondropathies and wherein said Adelmidrol is formulated in a formulation for parenteral administration by injection for infiltration into a joint, wherein Adelmidrol is contained in said formulations in a dosage from 1 mg to 7 g for the administration to a human with a body weight of about 70 kg or from 10 mg to 700 mg of the active ingredient by dose unit.

2. Adelmidrol for use according to claim 1, wherein said diseases are: articular chondropathies of mechanical, toxic, iatrogenic, degenerative origin or associated to inflammatory phenomena mainly related to organs and tissues not belonging to the osteoarticular system; fibrogenesis of the articular cartilages.

3. Adelmidrol for use according to claim 1 or 2, the said formulation consisting of:
- Adelmidrol 40 mg
- Hyaluronic acid sodium salt 20 mg
- Isosmotic solvent buffered to pH 7.0 q.s. to 5 mL.

## Patentansprüche

1. Adelmidrol zur Verwendung bei der Behandlung von Erkrankungen, die auf den spezifischen Agonismus des PPAR-gamma-Rezeptors ansprechen, bei Menschen oder Tieren, wobei es sich bei den Erkrankungen um artikuläre Chondropathien handelt und wobei das Adelmidrol in einer Formulierung zur parenteralen Verabreichung durch Injektion zur Infiltration in ein Gelenk formuliert ist, wobei Adelmidrol in den Formulierungen in einer Dosierung von 1 mg bis 7 g für die Verabreichung an einen Menschen mit einem Körpergewicht von etwa 70 kg oder von 10 mg bis 700 mg des Wirkstoffs pro Dosierungseinheit enthalten ist.

2. Adelmidrol zur Verwendung nach Anspruch 1, wobei es sich bei den Erkrankungen um Folgende handelt: artikuläre Chondropathien mechanischen, toxischen, iatrogenen oder degenerativen Ursprungs oder im Zusammenhang mit Entzündungsphänomenen, die hauptsächlich mit Organen und Geweben zusammenhängen, die nicht zum osteoartikulären System gehören; Fibrogenese der Gelenkknorpel.

3. Adelmidrol zur Verwendung nach Anspruch 1 oder 2, wobei die Formulierung besteht aus:
- Adelmidrol 40 mg
- Hyaluronsäure-Natriumsalz 20 mg
- Isosmotisches Lösungsmittel, gepuffert auf pH 7,0, q.s. bis 5 ml.

## Revendications

1. Adelmidrol pour une utilisation dans le traitement de maladies sensibles à l'agonisme spécifique du récepteur PPAR-gamma chez l'homme ou l'animal, lesdites maladies étant des chondropathies articulaires et ledit adelmidrol étant formulé dans une formulation pour administration parentérale par injection pour infiltration dans une articulation, l'adelmidrol étant contenu dans lesdites formulations à une dose de 1 mg à 7 g pour l'administration à un humain d'un poids corporel d'environ 70 kg ou de 10 mg à 700 mg de l'ingrédient actif par unité de dose.

2. Adelmidrol pour l'utilisation selon la revendication 1, dans lequel lesdites maladies sont : les chondropathies articulaires d'origine mécanique, toxique, iatrogène, dégénérative ou associées à des phénomènes inflammatoires principalement liés à des organes et tissus n'appartenant pas au système ostéoarticulaire ; la fibrogénèse des cartilages articulaires.

3. Adelmidrol pour l'utilisation selon la revendication 1 ou 2, ladite formulation étant constituée de :
- Adelmidrol 40 mg
- Sel de sodium de l'acide hyaluronique 20 mg
- Solvant isosmotique tamponné à un pH de 7,0 q.s. à 5 mL.
